(19) Europäisches Patentamt
European Patent Office
Office européen des brevets

(11) Veröffentlichungsnummer: **0 355 684 A1**

(12) # EUROPÄISCHE PATENTANMELDUNG

(21) Anmeldenummer: 89115082.3

(22) Anmeldetag: 16.08.89

(51) Int. Cl.4: **C07D 233/68 , A01N 43/50**

Patentansprüche für folgenden Vertragsstaat: ES.

(30) Priorität: 19.08.88 DE 3828208

(43) Veröffentlichungstag der Anmeldung: 28.02.90 Patentblatt 90/09

(84) Benannte Vertragsstaaten: BE CH DE ES FR GB IT LI

(71) Anmelder: BASF Aktiengesellschaft
Carl-Bosch-Strasse 38
D-6700 Ludwigshafen(DE)

(72) Erfinder: Wriede, Ulrich, Dr.
Birkenstrasse 7
D-6704 Mutterstadt(DE)
Erfinder: Hamprecht, Gerhard, Dr.
Rote-Turm-Strasse 28
D-6940 Weinheim(DE)
Erfinder: Koehler, Hermann, Dr.
In den Obstgaerten 7
D-6719 Bobenheim(DE)
Erfinder: Wuerzer, Bruno, Dr.
Ruedigerstrasse 13
D-6701 Otterstadt(DE)
Erfinder: Meyer, Norbert, Dr.
Dossenheimer Weg 22
D-6802 Ladenburg(DE)
Erfinder: Westphalen, Karl-Otto, Dr.
Mausbergweg 58
D-6720 Speyer(DE)

(54) **2-Chlorimidazolderivate, Verfahren zu ihrer Herstellung sowie sie enthaltende Mittel.**

(57) Substituierte 2-Chlorimidazolderivate Ia und Ib

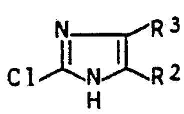 Ia

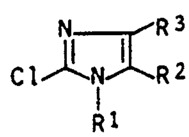 Ib

in denen die Substituenten folgende Bedeutung haben:
$R^1$ gegebenenfalls substituiertes $C_1$-$C_4$-Alkyl, $C_3$-$C_6$-Alkenyl oder $C_3$-$C_6$-Alkinyl;
$R^2$ gegebenenfils substituiertes $C_1$-$C_4$-Alkyl, $C_3$-$C_6$-Alkenyl, oder $C_3$-$C_6$-Alkinyl;
$R^3$ CN; $CO_2R^4$; $CONR^5R^6$ oder $COR^7$
$R^4$, $R^5$, $R^6$ und $R^7$ Wasserstoff; gegebenenfalls substituiertes $C_1$-$C_{10}$-Alkyl oder $C_3$-$C_{10}$-Cycloalkyl; $C_3$-$C_{10}$-Alkenyl, $C_3$-$C_{10}$-Alkinyl oder gegebenenfalls substituiertes Phenyl,
Verfahren zu ihrer Herstellung sowie sie enthaltende Mittel.

EP 0 355 684 A1

## 2-Chlorimidazolderivate, Verfahren zu ihrer Herstellung sowie sie enthaltende Mittel

Die Erfindung betrifft neue substituierte 2-Chlorimidazolderivate der Formeln Ia und Ib

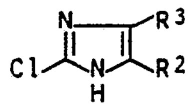

Ia

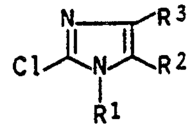

Ib

in denen die Substituenten folgende Bedeutung haben:
$R^1$ eine $C_1$-$C_4$-Alkylgruppe, eine $C_3$-$C_6$-Alkenylgruppe oder eine $C_3$-$C_6$-Alkinylgruppe, wobei diese Gruppen ein bis fünf Halogenatome und/oder einen Phenylrest tragen können;
$R^2$ eine $C_1$-$C_4$-Alkylgruppe, eine $C_3$-$C_6$-Alkenylgruppe, oder eine $C_3$-$C_6$-Alkinylgruppe, wobei diese Gruppen ein bis drei der folgenden Reste tragen können: Halogen, $C_1$-$C_4$-Alkoxy und/oder $C_1$-$C_4$-Alkylthio;
$R^3$ -CN; $-CO_2R^4$; $-CONR^5R^6$ oder $-COR^7$;
$R^4$, $R^5$, $R^6$ und $R^7$ Wasserstoff;
$C_1$-$C_{10}$-Alkyl, welche einfach durch $C_3$-$C_{10}$-Cycloalkyl, $C_1$-$C_4$-Alkoxy, $C_1$-$C_4$-Alkylthio oder Phenyl substituiert sein kann, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
$C_3$-$C_{10}$-Cycloalkyl, welches ein- bis dreifach durch $C_1$-$C_4$-Alkyl substituiert sein kann;
$C_3$-$C_{10}$-Alkenyl;
$C_3$-$C_{10}$-Alkinyl oder Phenyl, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
sowie deren umweltverträglichen Salze.

Die 2-Chlorimidazolderivate Ia können in verschiedenen tantomeren Formen vorliegen, die alle von der Formel Ia umfaßt werden sollen.

Außerdem betrifft die Erfindung ein Verfahren zur Herstellung der 2-Chlorimidazolderivate Ia und Ib sowie ihre Verwendung als Herbizide.

Aus der Literatur sind substituierte 2-Bromimidazol-4-carbonsäureester mit herbiziden Eigenschaften bekannt (EP-A 127 446; EP-A 180 787; US-A 4 711 962; US-A 4 591 377; US-A 4 578 106).

Besonders für die Anwendung neben Kulturpflanzen sind jedoch Verbindungen wünschenswert, die bei geringerer Aufwandmenge höhere Selektivität aufweisen.

Der Erfindung lag daher die Aufgabe zugrunde, Verbindungen zu synthetisieren, die diesen Ansprüchen genügen.

Entsprechend dieser Aufgabe wurden die eingangs definierten 2-Chlorimidazolderivate Ia und Ib gefunden. Weiterhin wurden Verfahren zur Herstellung dieser Verbindungen gefunden, und außerdem wurde gefunden, daß sie sich vorteilhaft als gegen Schadpflanzen selektiv wirkende Herbizide verwenden lassen.

Bisher in der Literatur beschriebene Verfahren zur Darstellung von 2-Chlorimidazolen, wie
- die Synthese über Halogenaustausch [US 3 499 606, J. Heterocyc. Chem. 4, 398 (1967)],
- die Chlorierung von Imidazolen mit N-Chlorsuccinimid [J. Heterocyc. Chem. 4, 451 (1967)] und
- die Metallierung von Imidazolen und anschließende Umsetzung mit N-Chlorsuccinimid [J. Org. Chem. 43, 4381 (1978)]

sind umständlich oder führen nur in schlechten Ausbeuten zum gewünschten Produkt.

Lediglich eine in J. Org. Chem., 28, 3041 (1963) beschriebene zweistufige Synthese liefert den 2-Chlorimidazoldicarbonsäurediester I′

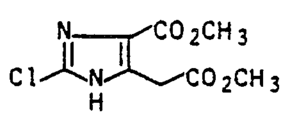

I′

in befriedigender Ausbeute.

Dazu wird in der ersten Stufe Acetondicarbonsäuredimethylester (II′) in wäßriger Essigsäure in situ

nitrosiert (IV'), reduziert (V') und cyclisiert (VII').

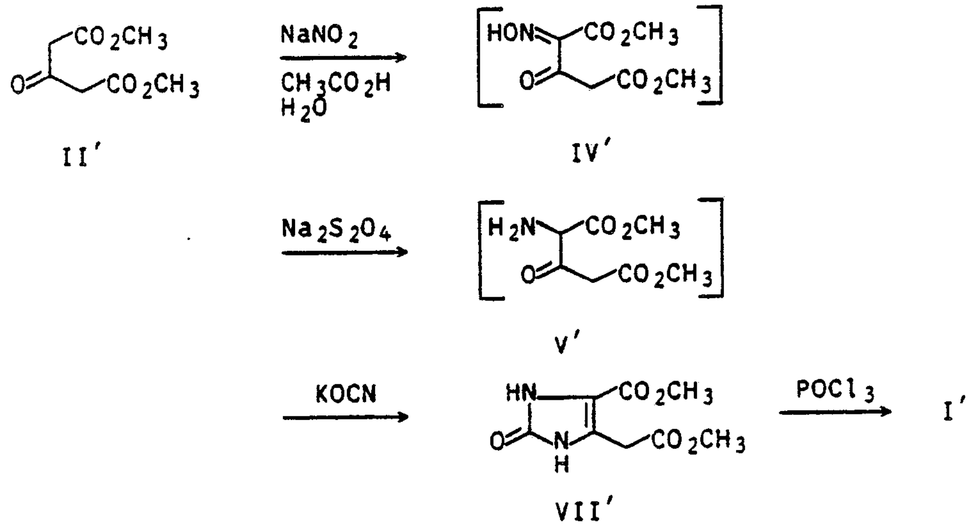


II' IV' V' VII' I'

Das so erhaltene Imidazolon VII' wird anschließend in Substanz mit Phosphoroxytrichlorid zum 2-Chlorimidazolderivat I' umgesetzt.

Das analoge Verfahren zur Darstellung der Verbindungen der Formeln Ia und/oder Ib zeigt einen breit anwendbaren Weg zu neuen substituierten 2-Chlorimidazolderivaten.

Auf diesem Wege können erstmals auch 2-Chlorimidazole mit empfindlichen Substituenten wie Amidogruppen oder Ketogruppen hergestellt werden.

Die Verbindungen der Formel Ia können nach zwei verschiedenen Verfahren hergestellt werden:

a) Analog zu der eingangs zitierten Methode kann eine Carbonylverbindung II in situ zu (IV) nitrosiert, zu (V) reduziert und anschließend zum Imidazolon VII cyclisiert werden.

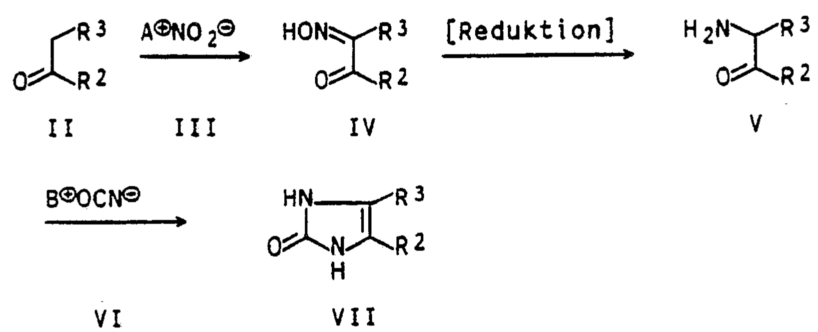


II III IV V

VI VII

Geeignete Nitrosierungsmittel sind beispielsweise Salze III in denen A$^{\oplus}$ ein Alkalimetallionen wie das Lithium-, das Natrium- und das Kaliumion bedeutet.

Als Reduktionsmittel dienen vorzugsweise anorganische Salze wie Natriumdithionit, Natriumhydrogensulfit, Natriumsulfit und Zinn-II-chlorid.

B$^{\oplus}$ bedeutet in Formel VI vorzugsweise das Ammonium-, Natrium- und Kaliumkation.

Diese Reaktionsfolge wird beispielsweise in einer wäßrigen Carbonsäure wie Ameisensäure, Essigsäure und Propionsäure oder in einer wäßrigen Mineralsäure wie Salzsäure und Schwefelsäure durchgeführt, wobei wäßrige Essigsäure und wäßrige Schwefelsäure bevorzugt werden.

Nitrosierung und Reduktion werden in der Regel bei Temperaturen von -10 bis 60 °C, vorzugsweise 20 bis 45 °C durchgeführt, während die Cyclisierung bei Temperaturen von 25 bis 100 °C, vorzugsweise 50 bis 100 °C abläuft.

Es kann vorteilhaft sein, vor der Cyclisierung der Reaktionsmischung ein inertes organisches Lösungsmittel (50 bis 150 Vol.%, vorzugsweise 80 bis 120 Vol.%) zuzusetzen.

Hier eignen sich beispielsweise Aceton, iso-Propanol, Tetrahydrofuran und Dioxan.

Die anschließende Umsetzung des Imidazolons VII mit einem Chlorierungsmittel IX liefert das Chlorimidazol Ia.

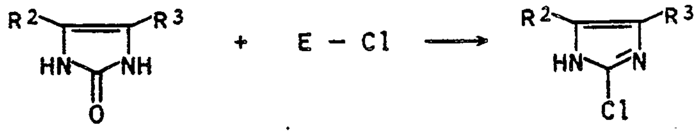

VII IX Ia

In der Formel IX bedeutet E den Rest eines gebräuchlichen Chlorierungsmittels. Geeignete Chlorierungsmittel sind z.B. Oxychloride wie Phosphoroxytrichlorid, Thionylchlorid oder Phosgen oder Chloride wie Phosphortrichlorid, Phosphorpentachlorid oder Schwefeltetrachlorid. Vorzugsweise verwendet man Phosphoroxytrichlorid.

Die Reaktion kann mit oder ohne Lösungsmittel bei Temperaturen von 25 bis 180 °C vorzugsweise 60 bis 160 °C, drucklos oder unter Druck (1 bis 10 bar, vorzugsweise 0 bis 3 bar), kontinuierlich oder diskontinuierlich gegebenenfalls in Anwesenheit einer Base durchgeführt werden.

Für diese Umsetzung geeignete Basen sind z.B. tertiäre Amine wie Triethylamin, Diisopropylethylamin, N,N-Dimethylanilin, N,N-Dimethyl-p-aminopyridin, Pyridin, Isochinolin, N-Methylpyrolidin, N,N,N′N′-Tetramethylethylendiamin, 1,5-Diazabicyclo(4,3,0)non-5-en und 1,8-Diazabicyclo(5,4,0)undec-7-en.

Als Lösungsmittel kommen beispielsweise Halogenkohlenwasserstoffe wie Methylenchlorid, Chloroform, Tetrachlorkohlenstoff, 1,1,2-Trichlorethan und Chlorbenzol; Ether wie Diethylether, Methyl-tert.-butylether, Ethylenglycoldimethylether, Tetrahydrofuran und Dioxan; Nitrokohlenwasserstoffe wie Nitrobenzol; Nitrile wie Acetonitril und Benzonitril und Kohlenwasserstoffe wie Hexan, Heptan, Cyclohexan, Dekalin, Ligroin oder Toluol in Betracht.

Vorzugsweise arbeitet man in Chlorbenzol oder Toluol oder ohne Lösungsmittel, mit Triethylamin oder Pyridin als Base oder ohne Base.

b) In einer vorteilhaften Variante des Verfahrens a) kann man die Verbindung IV aber auch mit einem inerten, nicht mit Wasser mischbaren organischen Lösungsmittel aus dem Reaktionsgemisch extrahieren und das isolierte Produkt in einer katalytischen Hydrierung an Edelmetall-Katalysatoren wie Platin oder Palladium oder nickelhaltigen Katalysatoren wie Raney-Nickel in einem inerten Lösungsmittel wie Essigsäure, Methanol, Ethanol oder Tetrahydrofuran bei 25 bis 50 °C, vorzugsweise 25 °C, unter Druck von 1 bis 100 bar, vorzugsweise 1 bis 50 bar, kontinuierlich oder diskontinuierlich mit Wasserstoff reduzieren.

Das so erhaltene Amin V kann ohne weitere Reinigung analog zu den unter a) beschriebenen Verfahren in das 2-Chlorimidazol Ia überführt werden.

Die Verbindungen Ib lassen sich aus den 2-Chlorimidazolen Ia in an sich bekannter Weise (Houben-Weyl, Bd. E5, S. 998 ff) durch Alkylierung, Alkenylierung oder Alkinylierung mit VIII erhalten.

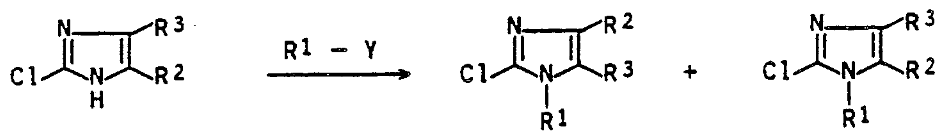

Ia VIII X Ib

In der Formel VIII bedeutet Y beispielsweise eine leicht abspaltbare Gruppe wie Halogen, z.B. Chlorid, Bromid und Iodid; Sulfonat wie Tosylat, Methylsulfonat und Trifluormethylsulfonat oder Alkylsulfat wie Methylsulfat und Ethylsulfat.

Die Umsetzung kann in einem inerten Lösungsmittel in Gegenwart einer starken Base bei Temperaturen von -25 bis 200 °C, vorzugsweise -10 bis 150 °C, drucklos oder unter Druck (1 bis 10 bar), kontinuierlich oder diskontinuierlich durchgeführt werden.

Als Basen eignen sich hierbei z.B. Alkaliverbindungen wie Natriumhydrid, Kaliumhydrid, Natriummethylat, Natriumethylat, Kalium-tert.butylat, Kaliumamid, Natrium- oder Kaliumhydroxid, Natriumcarbonat, Natriumbicarbonat und Kaliumcarbonat sowie die obengenannten tertiären Amine.

Bevorzugt werden Natriumhydrid, Natriummethylat, Kalium-tert.-butylat und 1,8-Diazabicyclo(5,4,0)-undec-7-en.

Geeignete Lösungsmittel sind z.B. Amide wie Dimethylformamid; Sulfoxide wie Dimethylsulfoxid; Ketone wie Aceton oder tert.-Butyl-methylketon und Alkohole wie Methanol, Ethanol und i-Propanol, sowie die vorstehend genannten Kohlenwasserstoffe und Ether.

Vorzugsweise wird die Reaktion in Tetrahydrofuran, Dimethylformamid, Toluol und/oder Dimethylsulfoxid durchgeführt.

Das als Nebenprodukt der Alkylierung anfallende Isomere X kann z.B. durch Chromatographie von den Verbindungen Ib abgetrennt werden.

In Hinblick auf die bestimmungsgemäße Verwendung der Verbindungen Ia und/oder Ib als Herbizide kommen als Substituenten folgende Reste in Betracht:

$R^1$ Alkyl wie Methyl, Ethyl, n-Propyl, iso-Propyl, n-Butyl, 1-Methylpropyl, 2-Methylpropyl und 1,1-Dimethylethyl, vorzugsweise Methyl, Ethyl und iso-Propyl;

Alkenyl wie Allyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1,2-Dimethyl-2-propenyl, 1,1-Dimethyl-2-propenyl, 1-Methyl-3-butenyl, 1-Ethyl-2-propenyl, 2-Hexenyl, 1-Methyl-2-pentenyl, 1-Ethyl-2-butenyl und 2-Ethyl-2-butenyl, vorzugsweise Allyl, 2-Butenyl und 1-Methyl-2-propenyl;

Alkinyl wie 2-Propinyl, 2-Butinyl, 3-Butinyl, 1-Methyl-2-propinyl, 1-Methyl-2-butinyl, 1-Ethyl-2-propinyl, 2-Pentinyl, 3-Pentinyl, 4-Pentinyl und 1-Methyl-2-pentinyl, vorzugsweise 2-Propinyl, 1-Methyl-2-propinyl und 2-Butinyl;

Halogenalkyl wie Trifluormethyl, Difluormethyl, Fluormethyl, Dichlormethyl, 1-Fluorethyl, 2-Fluorethyl und 2,2,2-Trifluorethyl, vorzugsweise Trifluormethyl, Difluormethyl und 2,2,2-Trifluorethyl oder

Phenylalkyl wie Benzyl, 1-Methylbenzyl, 2-Phenylethyl, 1-Methyl-2-phenylethyl, 2-Methyl-2-phenylethyl, 3-Phenylpropyl, 1-Methyl-2-phenylpropyl, 1-Methyl-3-phenylpropyl, 2-Methyl-2-phenylpropyl und 2-Methyl-3-phenylpropyl, vorzugsweise Benzyl, 1-Methylbenzyl und 2-Phenylethyl;

$R^2$ Alkyl wie unter $R^1$ genannt, insbesondere Methyl, Ethyl, Propyl und iso-Propyl;

Alkenyl wie unter $R^1$ genannt, insbesondere Allyl und 2-Butenyl;

Alkinyl wie unter $R^1$ genannt, insbesondere 2-Propinyl und 2-Butinyl,

wobei diese Gruppen ein bis drei der folgenden Reste tragen können:

Halogen wie Fluor, Chlor, Brom oder Iod, vorzugsweise Fluor und Chlor;

Alkoxy wie n-Propyloxy, iso-Propyloxy- und die isomeren Butoxyreste sowie vor allem die Methoxy- und die Ethoxygruppe und/oder

Alkylthio wie n-Propylthio, iso-Propylthio- und die isomeren Butylthioreste sowie vor allem die Methylthio- und die Ethylthiogruppe;

$R^3$ Cyano; $-CO_2R^4$; $-CONR^5R^6$ oder $COR^7$;

$R^4$, $R^5$, $R^6$ und $R^7$ Wasserstoff;

Alkyl, wie unter $R^1$ genannt, sowie n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimetylbutyl, 1,1,2-Trimethylpropyl, 1;2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, vorzugsweise Methyl, Ethyl, Propyl, i-Propyl, n-Butyl, iso-Butyl, 1-Methylbutyl, 1-Ethylpropyl, 1-Methylpropyl, 1-Ethylbutyl, n-Pentyl, 1-Methylpentyl, 1-Ethylpentyl, n-Hexyl, 1-Methylhexyl und 1-Ethylhexyl, welches einfach durch Cycloalkyl wie Cyclopropyl, Cyclobutyl, Cyclopentyl, Cyclohexyl, Cycloheptyl und der Cyclooctyl, vorzugsweise Cyclopropyl, Cyclobutyl, Cyclopentyl, Cyclohexyl und Cycloheptyl, Alkoxy oder Alkylthio wie im allgemeinen und im Besonderen bei $R^2$ genannt oder durch Phenyl substituiert sein kann, wobei der aromatische Ring seinerseits ein- bis fünffach durch Halogen wie insbesondere Fluor oder Chlor und/oder ein- bis dreifach durch Alkyl wie insbesondere bei $R^2$ genannt, Halogenalkyl wie insbesondere Difluromethyl und Trifluormethyl, Alkoxy wie insbesondere Difluormethoxy und Trifluormethoxy und/oder Alkylthio wie insbesondere Methylthio substituiert sein kann;

Cycloalkyl wie vorstehend genannt, welches ein- bis dreifach durch insbesondere Methyl und Ethyl substituiert sein kann;

Bei mehrfach substituierten Cycloalkylgruppen können alle möglichen Stereoisomeren vorkommen.

Alkenyl wie unter $R^1$ genannt, insbesondere Allyl, 1-Methyl-allyl, 2-Butenyl und 3-Butenyl;

Alkinyl wie unter $R^1$ genannt, insbesondere 2-Propinyl, 2-Butinyl, 2-Pentinyl und 1-Methyl-2-pentinyl und/oder Phenyl, wobei der aromatische Ringe seinerseits ein- bis dreifach durch insbesondere Difluromethyl und Trifluormethyl, Alkoxy wie insbesondere Difluormethoxy und Trifluormethoxy und/oder Alkylthio wie insbesondere Methylthio substituiert sein kann.

Die herbiziden 2-Chlorimidazolderivate Ia und Ib bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder

Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen Ia und Ib eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90% bis 100%, vorzugsweise 95% bis 100% (nach NMR-Spektrum) eingesetzt.

Die erfindungsgemäßen Verbindungen Ia und Ib können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.015 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.019 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.033 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.008 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 2.030 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 2.054 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.081 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 3,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |

| Botanischer Name | Deutscher Name |
|---|---|
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |

| Botanischer Name | Deutscher Name |
|---|---|
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die herbiziden 2-Chlorimidazolderivate Ia und Ib mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen Ia und Ib allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Beispielen wiedergegeben Vorschriften wurden unter entsprechender Abwandlung der Ausgangsverbindungen zur Gewinnung weiterer Verbindungen der Formel Ia und Ib benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellen und untersuchten Verbindungen eine gleichartige Wirkung erwarten.

Beispiel 1

2-Chlor-5-methylimidazol-4-carbonsäuremethylester

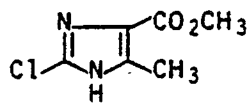

a) 79 g (0,6845 mol) Acetessigsäuremethylester in 150 ml Essigsäure wurden vorgelegt und so mit 40,2 g Natriumnitrit in 50 ml Wasser versetzt, daß die Temperatur 35° C nicht überstieg. Nach 30 Minuten wurden 550 ml Wasser zugesetzt, wonach eine Stunde bei Raumtemperatur nachgerührt wurde. Dann wurde eine Lösung aus 357 g Natriumdithionit in 600 ml Wasser zugesetzt und die Mischung mit verdünnter Schwefelsäure auf ca. pH 4 eingestellt. Anschließend wurden unter Eiskühlung portionsweise 186 g Kaliumcyanat zugegeben, 20 Minuten bei Raumtemperatur nachgerührt und die Mischung 5 min. auf 70° C erhitzt. Nach dem Abkühlen wurde der Niederschlag abfiltriert und getrocknet. Man erhielt 91 g (85 %) 5-Methyl-2-imidazolon-4-carbonsäuremethylester vom Fp: >225° C.

b) 83,1 g (0,5336 mol) 5-Methyl-2-imidazolon-4-carbonsäuremethylester und 800 ml Phosphoroxytrichlorid wurden 6 Stunden unter Rückfluß erhitzt. Überschüssiges Phosphoroxytrichlorid wurde bei vermindertem Druck abgezogen, der Rückstand auf Eiswasser gegeben und mit konzentrierter Ammoniaklösung auf pH 5 bis 6 eingestellt. Nach Extraktion mit Essigsäureethylester, Waschen und Trocknen erhielt man aus der organischen Phase ein Rohprodukt, welches nach chromatographischer Reinigung an Kieselgel mit Pentan/Essigester-Gemisch 54,9 g (59 %) 2-Chlor-5-methylimidazol-4-carbonsäuremethylester vom Fp: 149-150° C ergab (Wirkstoff-Nr. 1.001).

Beispiel 2

2-Chlor-5-methylimidazol-4-carbonsäureethylester

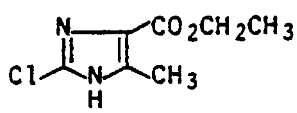

a) Aus 96,5 g (0,7356 mol) Acetessigsäureethylester in 160 ml Eisessig und 43,2 g Natriumnitrit in 64 ml Wasser erhielt man nach Reduktion mit 384 g Natriumdithionit analog zu Beispiel 1a) 87,5 g (70 %) 5-Methyl-2-imidazolon-4-carbonsäureethylester vom Fp.: 224-225° C.

b) 20 g (0,1176 mol) 5-Methyl-2-imidazolon-5-carbonsäureethylester und 320 ml Phosphoroxychlorid wurden analog zu Beispiel 1b) umgesetzt. Nach Chromatographie erhielt man 19,7 g (89 %) 2-Chlor-5-methylimidazol-4-carbonsäureethylester vom Fp.: 123-126° C (Wirkstoff Nr. 1.002)

Beispiel 3

2-Chlor-1,5-dimethylimidazol-4-carbonsäureethylester

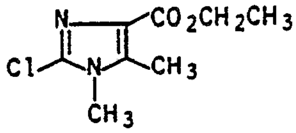

7,2 g (0,038 mol) 2-Chlor-5-methylimidazol-4-carbonsäureethylester (Beispiel 2b) wurden in 100 ml Tetrahydrofuran suspendiert, mit 1,2 g (0,052 mol) Natriumhydrid versetzt und gerührt, bis die Gasentwicklung beendet war. Anschließend wurde unter Kühlung mit 6,6 g (0,047 mol) Methyliodid versetzt und weitere 4 Stunden bei Raumtemperatur gerührt. Das Lösungsmittel wurde abdestilliert, der Rückstand auf Wasser gegeben und mit Essigester extrahiert, wonach die Essigesterphase gewaschen, getrocknet und eingeengt wurde. Die chromatographische Reinigung an Kieselgel (Laufmittel Pentan/Essigester) lieferte 5,8 g (75 %) 2-Chlor-1,5-dimethylimidazol-4-carbonsäureethylester vom Fp.: 80-82° C (Wirkstoff Nr. 2.008) und daneben noch geringe Mengen an 2-Chlor-1,4-dimethylimidazol-5-carbonsäureethylester vom Fp.: 61-63° C.

Tabelle 1

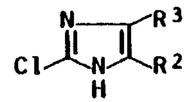

Ia

| Nr. | $R^3$ | $R^2$ | Fp (°C) | $^1H$-NMR (ppm) |
|---|---|---|---|---|
| 1.001 | $CO_2CH_3$ | $CH_3$ | 149-150 | |
| 1.002 | $CO_2CH_2CH_3$ | $CH_3$ | 123-126 | |
| 1.003 | $CO_2CH_2CH_2CH_3$ | $CH_3$ | 119-121 | |
| 1.004 | $CO_2CH(CH_3)_2$ | $CH_3$ | 160-165 | |
| 1.005 | $CO_2CH_2CH=CH_2$ | $CH_3$ | 134-135 | |
| 1.006 | $CO_2CH_2C\equiv CH$ | $CH_3$ | | |
| 1.007 | $CO_2C(CH_3)=CH_2$ | $CH_3$ | | |
| 1.008 | $CO_2CH_2$-cyclopropyl | $CH_3$ | | |
| 1.009 | $CO_2CH(CH_3)CH_2CH_3$ | $CH_3$ | 112-114 | |
| 1.010 | $CO_2CH_2CH_2CH_2CH_3$ | $CH_3$ | | |
| 1.011 | $CO_2CH_2$-cyclopropyl | $CH_3$ | | |
| 1.012 | $CO_2(CH_2)_4CH_3$ | $CH_3$ | | |
| 1.013 | $CO_2CH(CH_3)CH_2CH_2CH_3$ | $CH_3$ | 124-125 | |
| 1.014 | $CO_2CH(CH_2CH_3)_2$ | $CH_3$ | 147-148 | |
| 1.015 | $CO_2$- 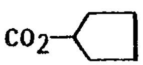 | $CH_3$ | 110-120 | |

Tabelle 1 (Fortsetzung)

| Nr. | $R^3$ | $R^2$ | Fp (°C) | $^1$H-NMR (ppm) |
|---|---|---|---|---|
| 1.016 | $CO_2CH_2$-cyclobutyl | $CH_3$ | | |
| 1.017 | $CO_2CH(CH_3)$-cyclopropyl | $CH_3$ | | |
| 1.018 | $CO_2CH(CH_3)(CH_2)_3CH_3$ | $CH_3$ | | |
| 1.019 | 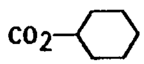 | $CH_3$ | 112-114 | |
| 1.020 | $CO_2CH(CH_3)(CH_2)_4CH_3$ | $CH_3$ | | |
| 1.021 | 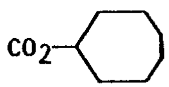 | $CH_3$ | | |
| 1.022 | 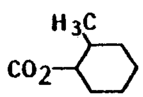 | $CH_3$ | | |
| 1.023 | 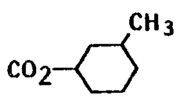 | $CH_3$ | | |
| 1.024 | 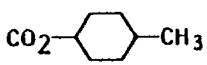 | $CH_3$ | | |
| 1.025 | 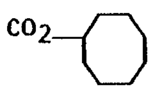 | $CH_3$ | | |

Tabelle 1 (Fortsetzung)

| Nr. | $R^3$ | $R^2$ | Fp (°C) | $^1$H-NMR (ppm) |
|---|---|---|---|---|
| 1.026 | $CO_2CH_3$ | $CH_2CH_3$ | 122-123 | |
| 1.027 | $CO_2CH_2CH_3$ | $CH_2CH_2CH_3$ | 119-120 | |
| 1.028 | $CO_2CH_3$ | $CH_2OCH_3$ | 119 | |
| 1.029 | $CO_2CH_2CH_3$ | $CF_3$ | 90-91 | |
| 1.030 | $CO_2CH(CH_3)_2$ | $CF_3$ | | |
| 1.031 | $CON(CH_3)_2$ | $CH_3$ | 115-117 | |
| 1.032 | C≡N | $C(CH_3)_3$ | 199-201 | |
| 1.033 | $COCH_3$ | $CH_3$ | 114-122 | |
| 1.034 | 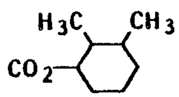 | $CH_3$ | | |
| 1.035 | 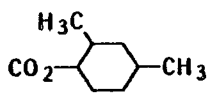 | $CH_3$ | | |
| 1.036 | 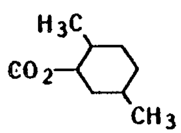 | $CH_3$ | | |

Tabelle 1 (Fortsetzung)

| Nr. | $R^3$ | $R^2$ | Fp (°C) | $^1$H-NMR (ppm) |
|---|---|---|---|---|
| 1.037 | 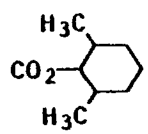 | $CH_3$ | | |
| 1.038 | 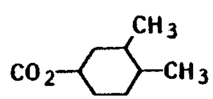 | $CH_3$ | | |
| 1.039 | 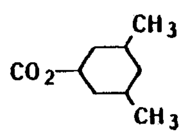 | $CH_3$ | | |

Tabelle 2

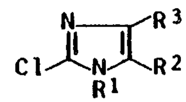

Ib

| Nr. | $R^3$ | $R^2$ | $R^1$ | Fp (°C) | $^1H$-NMR (ppm) |
|---|---|---|---|---|---|
| 2.001 | $CO_2CH_3$ | $CH_3$ | $CH_3$ | | |
| 2.002 | $CO_2CH_3$ | $CH_3$ | $CH_2CH_3$ | | |
| 2.003 | $CO_2CH_3$ | $CH_3$ | $CH(CH_3)_2$ | | |
| 2.004 | $CO_2CH_3$ | $CH_3$ | $CH_2CH{=}CH_2$ | | |
| 2.005 | $CO_2CH_3$ | $CH_3$ | $CH_2C{\equiv}CH$ | | |
| 2.006 | $CO_2CH_3$ | $CH_3$ | $CH_2CF_3$ | | |
| 2.007 | $CO_2CH_3$ | $CH_3$ | $CH_2C_6H_5$ | | |
| 2.008 | $CO_2CH_2CH_3$ | $CH_3$ | $CH_3$ | 80-82 | |
| 2.009 | $CO_2CH_2CH_3$ | $CH_3$ | $CH_2CH_3$ | | |
| 2.010 | $CO_2CH_2CH_3$ | $CH_3$ | $CH(CH_3)_2$ | | |
| 2.011 | $CO_2CH_2CH_3$ | $CH_3$ | $CH_2CH{=}CH_2$ | | |
| 2.012 | $CO_2CH_2CH_3$ | $CH_3$ | $CH_2C{\equiv}CH$ | | |
| 2.013 | $CO_2CH_2CH_3$ | $CH_3$ | $CH_2CF_3$ | | |
| 2.014 | $CO_2CH_2CH_3$ | $CH_3$ | $CH_2$-Ph | | |
| 2.015 | $CO_2CH_2CH_2CH_3$ | $CH_3$ | $CH_3$ | 89-90 | |
| 2.016 | $CO_2CH_2CH_2CH_3$ | $CH_3$ | $CH_2CH_3$ | | |
| 2.017 | $CO_2CH_2CH_2CH_3$ | $CH_3$ | $CH(CH_3)_2$ | | |
| 2.018 | $CO_2CH_2CH_2CH_3$ | $CH_3$ | $CH_2CH{=}CH_2$ | | 0,95(3H); 1,68(2H); 2,47(3H) 4,15(2H); 4,67(2H); 4,83(1H) 5,22(1H); 5,97(1H); |
| 2.019 | $CO_2CH_2CH_2CH_3$ | $CH_3$ | $CH_2C{\equiv}CH$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.020 | $CO_2CH_2CH_2CH_3$ | $CH_3$ | $CH_2CF_3$ | | |
| 2.021 | $CO_2CH_2CH_2CH_3$ | $CH_3$ | $CH_2$-Ph | | |
| 2.022 | $CO_2$-$CH(CH_3)_2$ | $CH_3$ | $CH_3$ | 87-88 | |
| 2.023 | $CO_2$-$CH(CH_3)_2$ | $CH_3$ | $CH_2CH_3$ | | 1.27(3H); 1.29(6H); 2.52(3H); 4.00(2H); 5.07(1H) |
| 2.024 | $CO_2$-$CH(CH_3)_2$ | $CH_3$ | $CH(CH_3)_2$ | | |
| 2.025 | $CO_2$-$CH(CH_3)_2$ | $CH_3$ | $CH_2CH=CH_2$ | | 1.27(6H); 2.47(3H); 4.63(2H); 4.80(1H) 5.08(1H); 5.22(1H); 5.97(1H) |
| 2.026 | $CO_2$-$CH(CH_3)_2$ | $CH_3$ | $CH_2C\equiv CH$ | | |
| 2.027 | $CO_2$-$CH(CH_3)_2$ | $CH_3$ | $CF_3$ | | |
| 2.028 | $CO_2$-$CH(CH_3)_2$ | $CH_3$ | $CH_2CF_3$ | | |
| 2.029 | $CO_2$-$CH(CH_3)_2$ | $CH_3$ | $CH_2$-Ph | | |
| 2.030 | $CO_2CH_2CH=CH_2$ | $CH_3$ | $CH_3$ | 45-46 | |
| 2.031 | $CO_2CH_2CH=CH_2$ | $CH_3$ | $CH(CH_3)_2$ | | |
| 2.032 | $CO_2CH_2CH=CH_2$ | $CH_3$ | $CF_3$ | | |
| 2.033 | $CO_2CH_2CH=CH_2$ | $CH_3$ | $CH_2$-Ph | | |
| 2.034 | $CO_2CH_2C\equiv CH$ | $CH_3$ | $CH_3$ | | |
| 2.035 | $CO_2CH_2C\equiv CH$ | $CH_3$ | $CH(CH_3)_2$ | | |
| 2.036 | $CO_2CH_2C\equiv CH$ | $CH_3$ | $CH_2$-Ph | | |
| 2.037 | $CO_2C(CH_3)=CH_2$ | $CH_3$ | $CH_3$ | | |
| 2.038 | $CO_2C(CH_3)=CH_2$ | $CH_3$ | $CH(CH_3)_2$ | | |
| 2.039 | $CO_2C(CH_3)=CH_2$ | $CH_3$ | $CF_3$ | | |
| 2.040 | $CO_2C(CH_3)=CH_2$ | $CH_3$ | $CH_2CF_3$ | | |
| 2.041 | $CO_2C(CH_3)=CH_2$ | $CH_3$ | $CH_2CH=CH_2$ | | |
| 2.042 | $CO_2C(CH_3)=CH_2$ | $CH_3$ | $CH_2C\equiv CH$ | | |
| 2.043 | $CO_2C(CH_3)=CH_2$ | $CH_3$ | $CH_2$-Ph | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.044 | $CO_2$-cyclopropyl | $CH_3$ | $CH_3$ | | |
| 2.045 | $CO_2$-cyclopropyl | $CH_3$ | $CH_2CH_3$ | | |
| 2.046 | $CO_2$-cyclopropyl | $CH_3$ | $CH_2CH_2CH_3$ | | |
| 2.047 | $CO_2$-cyclopropyl | $CH_3$ | $CH(CH_3)_2$ | | |
| 2.048 | $CO_2$-cyclopropyl | $CH_3$ | $CH_2CH=CH_2$ | | |
| 2.049 | $CO_2$-cyclopropyl | $CH_3$ | $CH_2C\equiv CH$ | | |
| 2.050 | $CO_2$-cyclopropyl | $CH_3$ | $CH_2C\equiv CCH_3$ | | |
| 2.051 | $CO_2$-cyclopropyl | $CH_3$ | $CF_3$ | | |
| 2.052 | $CO_2$-cyclopropyl | $CH_3$ | $CH_2CF_3$ | | |
| 2.053 | $CO_2$-cyclopropyl | $CH_3$ | $CH_2$-Ph | | |
| 2.054 | $CO_2CH(CH_3)CH_2CH_3$ | $CH_3$ | $CH_3$ | 58-60 | |
| 2.055 | $CO_2CH(CH_3)CH_2CH_3$ | $CH_3$ | $CH_2CH_3$ | | |
| 2.056 | $CO_2CH(CH_3)CH_2CH_3$ | $CH_3$ | $CH_2CH_2CH_3$ | | |
| 2.057 | $CO_2CH(CH_3)CH_2CH_3$ | $CH_3$ | $CH(CH_3)_2$ | | |
| 2.058 | $CO_2CH(CH_3)CH_2CH_3$ | $CH_3$ | $CH_2CH=CH_2$ | | |
| 2.059 | $CO_2CH(CH_3)CH_2CH_3$ | $CH_3$ | $CH_2C\equiv CH$ | | |
| 2.060 | $CO_2CH(CH_3)CH_2CH_3$ | $CH_3$ | $CF_3$ | | |
| 2.061 | $CO_2CH(CH_3)CH_2CH_3$ | $CH_3$ | $CH_2CF_3$ | | |
| 2.062 | $CO_2CH(CH_3)CH_2CH_3$ | $CH_3$ | $CH_2$-Ph | | |
| 2.063 | $CO_2CH_2CH_2CH_2CH_3$ | $CH_3$ | $CH_3$ | | |
| 2.064 | $CO_2CH_2CH_2CH_2CH_3$ | $CH_3$ | $CH(CH_3)_2$ | | |
| 2.065 | $CO_2CH_2CH_2CH_2CH_3$ | $CH_3$ | $CH_2CH=CH_2$ | | |
| 2.066 | $CO_2CH_2CH_2CH_2CH_3$ | $CH_3$ | $CH_2C\equiv CH$ | | |
| 2.067 | $CO_2CH_2CH_2CH_2CH_3$ | $CH_3$ | $CH_2$-Ph | | |
| 2.068 | $CO_2CH_2$-cyclopropyl | $CH_3$ | $CH_3$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.069 | $CO_2CH_2$-cyclopropyl | $CH_3$ | $CH_2CH_3$ | | |
| 2.070 | $CO_2CH_2$-cyclopropyl | $CH_3$ | $CH(CH_3)_2$ | | |
| 2.071 | $CO_2CH_2$-cyclopropyl | $CH_3$ | $CH_2CH=CH_2$ | | |
| 2.072 | $CO_2CH_2$-cyclopropyl | $CH_3$ | $CH_2C\equiv CH$ | | |
| 2.073 | $CO_2CH_2$-cyclopropyl | $CH_3$ | $CH_2$-Ph | | |
| 2.074 | $CO_2(CH_2)_4CH_3$ | $CH_3$ | $CH_3$ | | |
| 2.075 | $CO_2(CH_2)_4CH_3$ | $CH_3$ | $CH(CH_3)_2$ | | |
| 2.076 | $CO_2(CH_2)_4CH_3$ | $CH_3$ | $CH_2CH=CH_2$ | | |
| 2.077 | $CO_2(CH_2)_4CH_3$ | $CH_3$ | $CH_2C\equiv CH$ | | |
| 2.078 | $CO_2(CH_2)_4CH_3$ | $CH_3$ | $CF_3$ | | |
| 2.079 | $CO_2(CH_2)_4CH_3$ | $CH_3$ | $CH_2CF_3$ | | |
| 2.080 | $CO_2(CH_2)_4CH_3$ | $CH_3$ | $CH_2$-Ph | | |
| 2.081 | $CO_2CH(CH_3)CH_2CH_2CH_3$ | $CH_3$ | $CH_3$ | | 0.90(3H); 1.26(3H); 1.33(2H); 1.58(2H); 2.48(3H); 3.52(3H); 5.00(1H) |
| 2.082 | $CO_2CH(CH_3)CH_2CH_2CH_3$ | $CH_3$ | $CH_2CH_3$ | | |
| 2.083 | $CO_2CH(CH_3)CH_2CH_2CH_3$ | $CH_3$ | $CH_2CH_2CH_3$ | | |
| 2.084 | $CO_2CH(CH_3)CH_2CH_2CH_3$ | $CH_3$ | $CH(CH_3)_2$ | | |
| 2.085 | $CO_2CH(CH_3)CH_2CH_2CH_3$ | $CH_3$ | $CH_2CH(CH_3)_2$ | | |
| 2.086 | $CO_2CH(CH_3)CH_2CH_2CH_3$ | $CH_3$ | $CF_3$ | | |
| 2.087 | $CO_2CH(CH_3)CH_2CH_2CH_3$ | $CH_3$ | $CH_2CF_3$ | | |
| 2.088 | $CO_2CH(CH_3)CH_2CH_2CH_3$ | $CH_3$ | $CH_2CH=CH_2$ | | 0.92(3H); 1.25(3H); 1.32(2H); 1.75(2H); 2.47(3H); 4.63(2H); 4.85(1H); 5.02(1H); 5.22(1H); 5.97(1H); |

Tabelle 2 (Fortsetzung)

| Nr. | $R^3$ | $R^2$ | $R^1$ | Fp (°C) | $^1$H-NMR (ppm) |
|---|---|---|---|---|---|
| 2.089 | $CO_2CH(CH_3)CH_2CH_2CH_3$ | $CH_3$ | $CH_2C{\equiv}CH$ | | |
| 2.090 | $CO_2CH(CH_3)CH_2CH_2CH_3$ | $CH_3$ | $CH_2$-Ph | | |
| 2.091 | $CO_2CH(CH_2CH_3)_2$ | $CH_3$ | $CH_3$ | | 1.37(6H); 1.62(4H); 2.48(3H); 4.84(1H) |
| 2.092 | $CO_2CH(CH_2CH_3)_2$ | $CH_3$ | $CH_2CH_3$ | | |
| 2.093 | $CO_2CH(CH_2CH_3)_2$ | $CH_3$ | $CH_2CH_2CH_3$ | | |
| 2.094 | $CO_2CH(CH_2CH_3)_2$ | $CH_3$ | $CH(CH_3)_2$ | | |
| 2.095 | $CO_2CH(CH_2CH_3)_2$ | $CH_3$ | $CH_2CH(CH_3)_2$ | | |
| 2.096 | $CO_2CH(CH_2CH_3)_2$ | $CH_3$ | $CH(CH_3)CH_2CH_3$ | | |
| 2.097 | $CO_2CH(CH_2CH_3)_2$ | $CH_3$ | $CF_3$ | | |
| 2.098 | $CO_2CH(CH_2CH_3)_2$ | $CH_3$ | $CH_2CF_3$ | | |
| 2.099 | $CO_2CH(CH_2CH_3)_2$ | $CH_3$ | $CH_2CH{=}CH_2$ | | |
| 2.100 | $CO_2CH(CH_2CH_3)_2$ | $CH_3$ | $CH_2C{\equiv}CH$ | | |
| 2.101 | $CO_2CH(CH_2CH_3)_2$ | $CH_3$ | $CH_2$-Ph | | |
| 2.102 | $CO_2$- 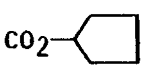 | $CH_3$ | $CH_3$ | 67-68 | |
| 2.103 | $CO_2$- 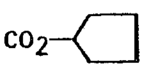 | $CH_3$ | $CH_2CH_3$ | | 1.23(3H); 1.70(6H); 1.90(2H); 2.50(3H); 3.98(2H); 5.23(1H); |
| 2.104 | $CO_2$- 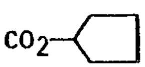 | $CH_3$ | $CH_2CH_2CH_3$ | | |

Tabelle 2 (Fortsetzung)

| Nr. | $R^3$ | $R^2$ | $R^1$ | Fp (°C) | $^1H$-NMR (ppm) |
|---|---|---|---|---|---|
| 2.105 | $CO_2$– 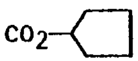 | $CH_3$ | $CH(CH_3)_2$ | | 1.48(6H); 1.65(6H); 1.88(2H); 2.56(3H); 4.60(1H); 5.22(1H); |
| 2.106 | $CO_2$– 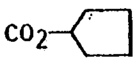 | $CH_3$ | $CH_2CH=CH_2$ | | 1,60-1,91(8H); 2,46(3H); 4,67(2H); 4,82(1H); 5,23(d,1H); 5,25(1H); 5,97(1H) |
| 2.107 | $CO_2$– 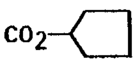 | $CH_3$ | $CH_2C≡CH$ | 88-89 | |
| 2.108 | $CO_2$– 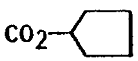 | $CH_3$ | $CF_3$ | | |
| 2.109 | $CO_2$– 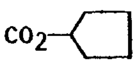 | $CH_3$ | $CH_2CF_3$ | | |
| 2.110 | $CO_2$– 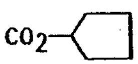 | $CH_3$ | $CH_2$-Ph | | |
| 2.111 | $CO_2CH_2$-cyclobutyl | $CH_3$ | $CH_3$ | | |
| 2.112 | $CO_2CH_2$-cyclobutyl | $CH_3$ | $CH_2CH_3$ | | |
| 2.113 | $CO_2CH_2$-cyclobutyl | $CH_3$ | $CH(CH_3)_2$ | | |
| 2.114 | $CO_2CH_2$-cyclobutyl | $CH_3$ | $CH_2C=CH_2$ | | |
| 2.115 | $CO_2CH_2$-cyclobutyl | $CH_3$ | $CH_2C≡CH$ | | |
| 2.116 | $CO_2CH_2$-cyclobutyl | $CH_3$ | $CH_2$-Ph | | |
| 2.117 | $CO_2CH(CH_3)$-cyclopropyl | $CH_3$ | $CH_3$ | | |
| 2.118 | $CO_2CH(CH_3)$-cyclopropyl | $CH_3$ | $CH_2CH_3$ | | |
| 2.119 | $CO_2CH(CH_3)$-cyclopropyl | $CH_3$ | $CH(CH_3)_2$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.120 | $CO_2CH(CH_3)$-cyclopropyl | $CH_3$ | $CH_2CH=CH_2$ | | |
| 2.121 | $CO_2CH(CH_3)$-cyclopropyl | $CH_3$ | $CH_2C\equiv CH$ | | |
| 2.122 | $CO_2CH(CH_3)$-cyclopropyl | $CH_3$ | $CH_2$-Ph | | |
| 2.123 | $CO_2CH(CH_3)(CH_2)_3CH_3$ | $CH_3$ | $CH_3$ | | |
| 2.124 | $CO_2CH(CH_3)(CH_2)_3CH_3$ | $CH_3$ | $CH(CH_3)_2$ | | |
| 2.125 | $CO_2CH(CH_3)(CH_2)_3CH_3$ | $CH_3$ | $CH_2CH=CH_2$ | | |
| 2.126 | $CO_2CH(CH_3)(CH_2)_3CH_3$ | $CH_3$ | $CH_2C\equiv CH$ | | |
| 2.127 | $CO_2CH(CH_3)(CH_2)_3CH_3$ | $CH_3$ | $CH_2$-Ph | | |
| 2.128 | $CO_2$ 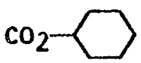 | $CH_3$ | $CH_3$ | 95-97 | |
| 2.129 | $CO_2$ 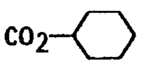 | $CH_3$ | $CH_2CH_3$ | | 1,24 (t, 3H); 1,33-1,87 (m, 10H); 2,53 (s, 3H); 3,98 (g, 2H); 4,85 (m, 1H) |
| 2.130 | $CO_2$ 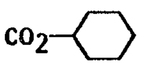 | $CH_3$ | $CH_2CH_2CH_3$ | | |
| 2.131 | $CO_2$ 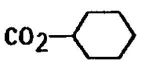 | $CH_3$ | $CH(CH_3)_2$ | | |
| 2.132 | $CO_2$ 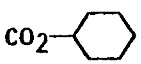 | $CH_3$ | $CH_2CH(CH_3)_2$ | | |

Tabelle 2 (Fortsetzung)

| Nr. | $R^3$ | $R^2$ | $R^1$ | Fp (°C) | $^1H$-NMR (ppm) |
|---|---|---|---|---|---|
| 2.133 | $CO_2$- 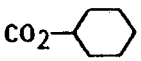 | $CH_3$ | $CH(CH_3)CH_2CH_3$ | | |
| 2.134 | $CO_2$- 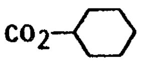 | $CH_3$ | $CH_2CH=CH_2$ | | 1.38(6H); 1.72(2H); 1.88(2H); 2.47(3H); 4.64(2H); 4.80(1H); 4.85(1H); 5.22(1H); 5.97(1H) |
| 2.135 | $CO_2$- 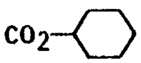 | $CH_3$ | $CH_2C{\equiv}CH$ | | 1.40(6H); 1.70(2H); 1.87(2H); 2.55(3H); 3.57(1H); 4.83(1H); 4.92(2H) |
| 2.136 | $CO_2$- 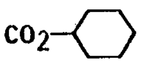 | $CH_3$ | $CH_2$-Ph | | |
| 2.137 | $CO_2$- 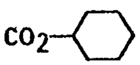 | $CH_3$ | $CF_3$ | | |
| 2.138 | $CO_2$- 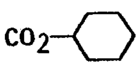 | $CH_3$ | $CH_2CF_3$ | | |
| 2.139 | $CO_2CH(CH_3)(CH_2)_4CH_3$ | $CH_3$ | $CH_3$ | | |
| 2.140 | $CO_2CH(CH_3)(CH_2)_4CH_3$ | $CH_3$ | $CH(CH_3)_2$ | | |
| 2.141 | $CO_2CH(CH_3)(CH_2)_4CH_3$ | $CH_3$ | $CH_2CH=CH_2$ | | |
| 2.142 | $CO_2CH(CH_3)(CH_2)_4CH_3$ | $CH_3$ | $CH_2C{\equiv}CH$ | | |
| 2.143 | $CO_2CH(CH_3)(CH_2)_4CH_3$ | $CH_3$ | $CH_2$-Ph | | |
| 2.144 | $CO_2$- 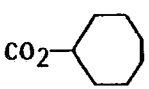 | $CH_3$ | $CH_3$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.145 | 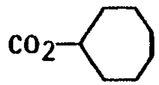 | $CH_3$ | $CH_2CH_3$ | | |
| 2.146 | 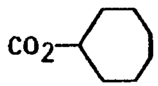 | $CH_3$ | $CH_2CF_3$ | | |
| 2.147 | 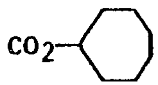 | $CH_3$ | $CH_2CH_2CH_3$ | | |
| 2.148 | 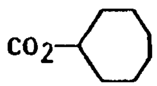 | $CH_3$ | $CH(CH_3)_2$ | | |
| 2.149 | 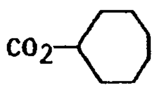 | $CH_3$ | $CH_2CH{=}CH_2$ | | |
| 2.150 | 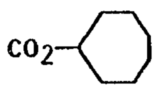 | $CH_3$ | $CH_2C{\equiv}CH$ | | |
| 2.151 | 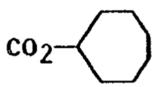 | $CH_3$ | $CH_2$-Ph | | |
| 2.152 | 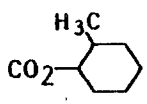 | $CH_3$ | $CH_3$ | | |
| 2.153 | 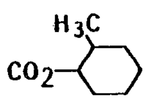 | $CH_3$ | $CH_2CH_3$ | | |

Tabelle 2 (Fortsetzung)

| Nr. | $R^3$ | $R^2$ | $R^1$ | Fp (°C) | $^1H$-NMR (ppm) |
|---|---|---|---|---|---|
| 2.154 | 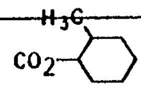 | $CH_3$ | $CH_2CH_2CH_3$ | | |
| 2.155 | 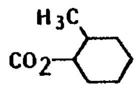 | $CH_3$ | $CH(CH_3)_2$ | | |
| 2.156 | 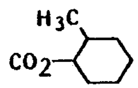 | $CH_3$ | $CH_2CH{=}CH_2$ | | |
| 2.157 | 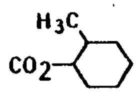 | $CH_3$ | $CH_2C{\equiv}CH$ | | |
| 2.158 | 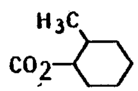 | $CH_3$ | $CH_2$-Ph | | |
| 2.159 | 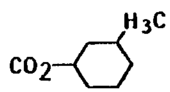 | $CH_3$ | $CH_3$ | | |
| 2.160 | 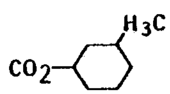 | $CH_3$ | $CH_2CH_3$ | | |
| 2.161 | 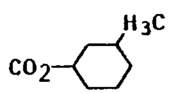 | $CH_3$ | $CH_2CH_2CH_3$ | | |
| 2.162 | 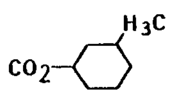 | $CH_3$ | $CH(CH_3)_2$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.163 | $CO_2$ 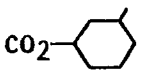 | $CH_3$ | $CH_2CH=CH_2$ | | |
| 2.164 | $CO_2$ 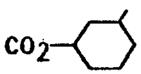 | $CH_3$ | $CH_2C\equiv CH$ | | |
| 2.165 | $CO_2$ 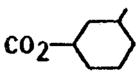 | $CH_3$ | $CH_2$-Ph | | |
| 2.166 | $CO_2$ 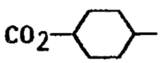 | $CH_3$ | $CH_3$ | | |
| 2.167 | $CO_2$ 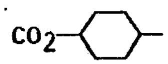 | $CH_3$ | $CH_2CH_3$ | | |
| 2.168 | $CO_2$ 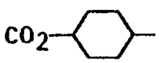 | $CH_3$ | $CH(CH_3)_2$ | | |
| 2.169 | $CO_2$ 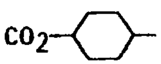 | $CH_3$ | $CH_2CH=CH_2$ | | |
| 2.170 | $CO_2$ 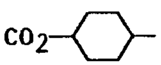 | $CH_3$ | $CH_2C\equiv CH$ | | |
| 2.171 | $CO_2$ 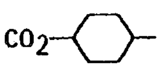 | $CH_3$ | $CH_2$-Ph | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.172 | $CO_2$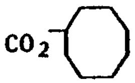 | $CH_3$ | $CH_3$ | | |
| 2.173 | $CO_2$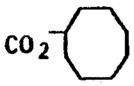 | $CH_3$ | $CH_2CH_3$ | | |
| 2.174 | $CO_2$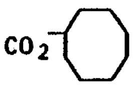 | $CH_3$ | $CH(CH_3)_2$ | | |
| 2.175 | $CO_2$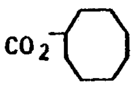 | $CH_3$ | $CH_2CH=CH_2$ | | |
| 2.176 | $CO_2$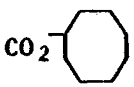 | $CH_3$ | $CH_2C\equiv CH$ | | |
| 2.177 | $CO_2$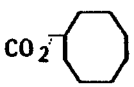 | $CH_3$ | $CH_2$-Ph | | |
| 2.178 | $CO_2CH_3$ | $CH_2CH_3$ | $CH_3$ | | 1.13(t)3H;2.94 (q)2H;3.59(s)3H;3.66(s) 3H |
| 2.179 | $CO_2CH_3$ | $CH_2CH_3$ | $CH(CH_3)_2$ | | |
| 2.180 | $CO_2CH_3$ | $CH_2CH_3$ | $CH_2CH=CH_2$ | | |
| 2.181 | $CO_2CH_3$ | $CH_2CH_3$ | $CH_2C\equiv CH$ | | |
| 2.182 | $CO_2CH_3$ | $CH_2CH_3$ | $CH_2$-Ph | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.183 | $CO_2CH_2CH_3$ | $CH_2CH_2CH_3$ | $CH_3$ | | 0.93(t) 3H;1.29(t)3H;1.53(q) 2H;2.91(t)2H;3.57(s) 3H;4.22(q)2H |
| 2.184 | $CO_2CH_2CH_3$ | $CH_2CH_2CH_3$ | $CH(CH_3)_2$ | | |
| 2.185 | $CO_2CH_2CH_3$ | $CH_2CH_2CH_3$ | $CH_2CH{=}CH_2$ | | |
| 2.186 | $CO_2CH_2CH_3$ | $CH_2CH_2CH_3$ | $CH_2C{\equiv}CH$ | | |
| 2.187 | $CO_2CH_2CH_3$ | $CH_2CH_2CH_3$ | $CH_2$-Ph | | |
| 2.188 | $CO_2CH_3$ | $CH_2OCH_3$ | $CH_3$ | | |
| 2.189 | $CO_2CH_3$ | $CH_2OCH_3$ | $CH(CH_3)_2$ | | |
| 2.190 | $CO_2CH_3$ | $CH_2OCH_3$ | $CH_2$-Ph | | |
| 2.191 | $CO_2CH_2CH_3$ | $CF_3$ | $CH_3$ | 62-63 | |
| 2.192 | $CO_2CH_2CH_3$ | $CF_3$ | $CH(CH_3)_2$ | | |
| 2.193 | $CO_2CH_2CH_3$ | $CF_3$ | $CH_2CH{=}CH_2$ | | |
| 2.194 | $CO_2CH_2CH_3$ | $CF_3$ | $CH_2C{\equiv}CH$ | | |
| 2.195 | $CO_2CH_2CH_3$ | $CF_3$ | $CH_2$-Ph | | |
| 2.196 | $CO_2CH(CH_3)_2$ | $CF_3$ | $CH_3$ | | |
| 2.197 | $CO_2CH(CH_3)_2$ | $CF_3$ | $CH(CH_3)_2$ | | |
| 2.198 | $CO_2CH(CH_3)_2$ | $CF_3$ | $CH_2$-Ph | | |
| 2.199 | $CON(CH_3)_2$ | $CH_3$ | $CH_3$ | | |
| 2.200 | $CON(CH_3)_2$ | $CH_3$ | $CH(CH_3)_2$ | | |
| 2.201 | $CON(CH_3)_2$ | $CH_3$ | $CH_2$-Ph | | |
| 2.202 | $C{\equiv}N$ | $C(CH_3)_3$ | $CH_3$ | | |
| 2.203 | $C{\equiv}N$ | $C(CH_3)_3$ | $CH(CH_3)_2$ | | |

Tabelle 2 (Fortsetzung)

| Nr. | $R^3$ | $R^2$ | $R^1$ | Fp (°C) | $^1H$-NMR (ppm) |
|---|---|---|---|---|---|
| 2.204 | C≡N | $C(CH_3)_3$ | $CH_2CH=CH_2$ | | |
| 2.205 | C≡N | $C(CH_3)_3$ | $CH_2C≡CH$ | | |
| 2.206 | C≡N | $C(CH_3)_3$ | $CF_3$ | | |
| 2.207 | C≡N | $C(CH_3)_3$ | $CH_2CF_3$ | | |
| 2.208 | C≡N | $C(CH_3)_3$ | $CH_2$-Ph | | |
| 2.209 | $COCH_3$ | $CH_3$ | $CH_3$ | | |
| 2.210 | $COCH_3$ | $CH_3$ | $CH(CH_3)_2$ | | |
| 2.211 | $COCH_3$ | $CH_3$ | $CH_2CH=CH_2$ | | |
| 2.212 | $COCH_3$ | $CH_3$ | $CH_2$-Ph | | |
| 2.213 | 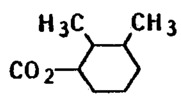 | $CH_3$ | $CH_3$ | | |
| 2.214 | 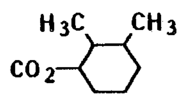 | $CH_3$ | $CH_2CH_2$ | | |
| 2.215 | 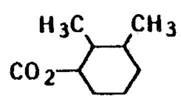 | $CH_3$ | $CH(CH_3)_2$ | | |
| 2.216 | 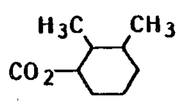 | $CH_3$ | $CH_2CH=CH_2$ | | |

Tabelle 2 (Fortsetzung)

| Nr. | $R^3$ | $R^2$ | $R^1$ | Fp (°C) | $^1H$-NMR (ppm) |
|---|---|---|---|---|---|
| 2.217 | 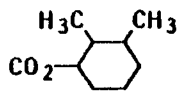 | $CH_3$ | $CH_2C{\equiv}CH$ | | |
| 2.218 | 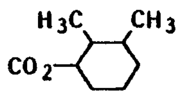 | $CH_3$ | $CH_2Ph$ | | |
| 2.219 | 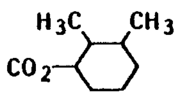 | $CH_3$ | $CF_3$ | | |
| 2.220 | 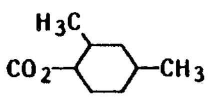 | $CH_3$ | $CH_3$ | | |
| 2.221 | 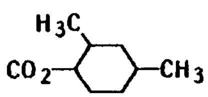 | $CH_3$ | $CH_2CH_3$ | | |
| 2.222 | 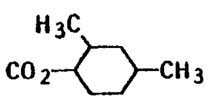 | $CH_3$ | $CH(CH_3)_2$ | | |
| 2.223 | 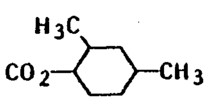 | $CH_3$ | $CH_2CH=CH_2$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.224 | 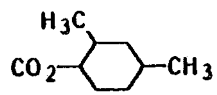 | $CH_3$ | $CH_2C{\equiv}CH$ | | |
| 2.225 | 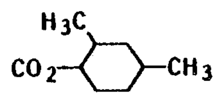 | $CH_3$ | $CH_2Ph$ | | |
| 2.226 | 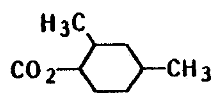 | $CH_3$ | $CF_3$ | | |
| 2.227 | 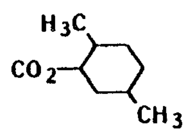 | $CH_3$ | $CH_3$ | | |
| 2.228 | 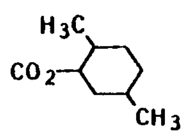 | $CH_3$ | $CH_2CH_3$ | | |
| 2.229 | 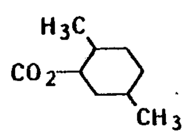 | $CH_3$ | $CH(CH_3)_2$ | | |

Tabelle 2 (Fortsetzung)

| Nr. | $R^3$ | $R^2$ | $R^1$ | Fp (°C) | $^1H$-NMR (ppm) |
|---|---|---|---|---|---|
| 2.230 | 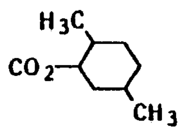 | $CH_3$ | $CH_2CH{=}CH_2$ | | |
| 2.231 | 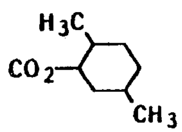 | $CH_3$ | $CH_2C{\equiv}CH$ | | |
| 2.232 | 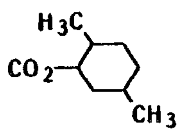 | $CH_3$ | $CH_2Ph$ | | |
| 2.233 | 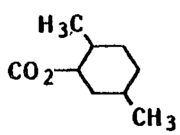 | $CH_3$ | $CF_3$ | | |
| 2.234 | 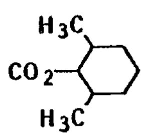 | $CH_3$ | $CH_3$ | | |
| 2.235 | 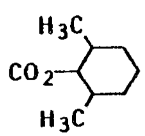 | $CH_3$ | $CH_2CH_3$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.236 | 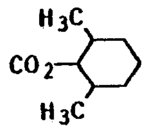 | $CH_3$ | $CH(CH_3)_2$ | | |
| 2.237 | 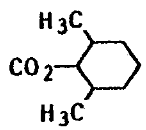 | $CH_3$ | $CH_2CH=CH_2$ | | |
| 2.238 | 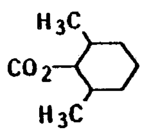 | $CH_3$ | $CH_2C\equiv CH$ | | |
| 2.239 | 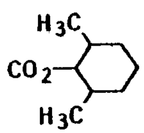 | $CH_3$ | $CH_2Ph$ | | |
| 2.240 | 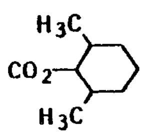 | $CH_3$ | $CF_3$ | | |
| 2.241 | 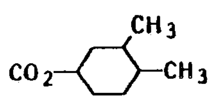 | $CH_3$ | $CH_3$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.242 | 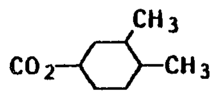 | $CH_3$ | $CH_2CH_3$ | | |
| 2.243 | 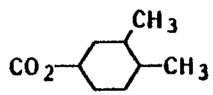 | $CH_3$ | $CH(CH_3)_2$ | | |
| 2.244 | 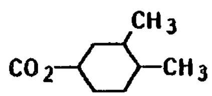 | $CH_3$ | $CH_2CH=CH_2$ | | |
| 2.245 | 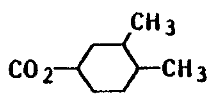 | $CH_3$ | $CH_2C\equiv CH$ | | |
| 2.246 | 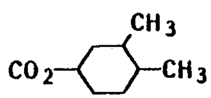 | $CH_3$ | $CH_2Ph$ | | |
| 2.247 | 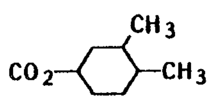 | $CH_3$ | $CF_3$ | | |
| 2.248 | 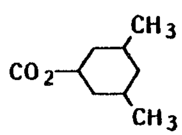 | $CH_3$ | $CH_3$ | | |

Tabelle 2 (Fortsetzung)

| Nr. | $R^3$ | $R^2$ | $R^1$ | Fp (°C) | $^1$H-NMR (ppm) |
|---|---|---|---|---|---|
| 2.249 | 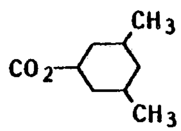 | $CH_3$ | $CH_2CH_3$ | | |
| 2.250 | 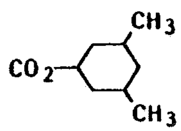 | $CH_3$ | $CH(CH_3)_2$ | | |
| 2.251 | 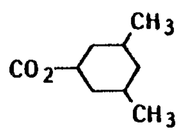 | $CH_3$ | $CH_2CH=CH_2$ | | |
| 2.252 | 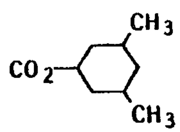 | $CH_3$ | $CH_2C\equiv CH$ | | |
| 2.253 | 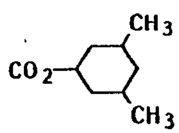 | $CH_3$ | $CH_2Ph$ | | |
| 2.254 | 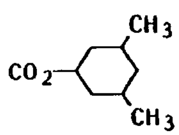 | $CH_3$ | $CF_3$ | | |

Anwendungsbeispiele

Die herbizide Wirkung der 2-Chlorimidazole Ia und Ib ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewaschsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmengen für die Nachauflaufbehandlung betrugen 0,25 kg Wirkstoff/ha. Eine Abdeckung unterblieb ber der Nachauflaufbehandlung.

Die Versuchsgefäße wurden im Gewächshaus aufgestellt, wobei für wärmeliebende Arten wärmere Bereiche (20 bis 35 °C) und für solche gemäßigter Klimate 10 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name | Englischer Name |
|---|---|---|---|
| SOLNI | Solanum nigrum | Schwarzer Nachtschatten | black nightshade |
| STEME | Stellaria media | Vogelsternmiere | chickweed |
| VERSS | Veronica spp. | Ehrenpreisarten | speedwell |
| ZEAMX | Zea mays | Mais | Indian corn |

Mit 0,25 kg/ha a.S. im Nachauflaufverfahren eingesetzt, lassen sich mit den Beispielen 2.022 und 2.054 breitblättrige unerwünschte Pflanzen sehr gut bekämpfen. Bei Vorauflaufanwendung mit 0,25 kg/ha a.S. werden mit den Beispielen 2.022 und 2.054 breitblättrige unerwünschte Pflanzen gut bekämpft, bei gleichzeitiger guter Verträglichkeit für eine Beispielkultur.

## Ansprüche

1. Substituierte 2-Chlorimidazolderivate der Formeln Ia und Ib

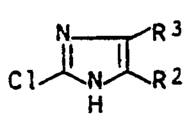 Ia 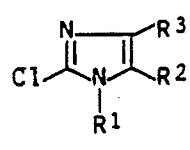 Ib

in denen die Substituenten folgende Bedeutung haben:

$R^1$ eine $C_1$-$C_4$-Alkylgruppe, eine $C_3$-$C_6$-Alkenylgruppe oder eine $C_3$-$C_6$-Alkinylgruppe, wobei diese Gruppen ein bis fünf Halogenatome und/oder einen Phenylrest tragen können;

$R^2$ eine $C_1$-$C_4$-Alkylgruppe, eine $C_3$-$C_6$-Alkenylgruppe, oder eine $C_3$-$C_6$-Alkinylgruppe, wobei diese Gruppen

ein bis drei der folgenden Reste tragen können: Halogen, $C_1$-$C_4$-Alkoxy und/oder $C_1$-$C_4$-Alkylthio;
$R^3$ -CN; -$CO_2R^4$; -$CONR^5R^6$ oder -$COR^7$;
$R^4$, $R^5$, $R^6$ und $R^7$ Wasserstoff;
$C_1$-$C_{10}$-Alkyl, welche einfach durch $C_3$-$C_{10}$-Cycloalkyl, $C_1$-$C_4$-Alkoxy, $C_1$-$C_4$-Alkylthio oder Phenyl substituiert sein kann, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
$C_3$-$C_{10}$-Cycloalkyl, welches ein- bis dreifach durch $C_1$-$C_4$-Alkyl substituiert sein kann;
$C_3$-$C_{10}$-Alkenyl;
$C_3$- $C_{10}$-Alkinyl oder Phenyl, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
sowie deren umweltverträglichen Salze.

2. Verfahren zur Herstellung der Verbindungen Ia gemäß Anspruch 1, dadurch gekennzeichnet, daß man eine Carbonylverbindung II

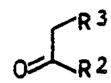

II

in an sich bekannter Weise ohne Isolierung der Zwischenstufen nacheinander in einer wäßrigen Säure zuerst mit einem Nitrit III,
$A^{\oplus}NO_2^{\ominus}$ III
worin $A^{\oplus}$ ein Alkalimetallion bedeutet, zu IV nitrosiert,

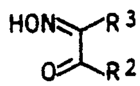

IV

danach zu V reduziert,

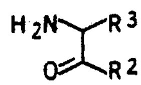

V

und direkt mit einem Cyanat VI,
$B^{\oplus}OCN^{\ominus}$ VI
worin $B^{\oplus}$ für ein Alkalimetallkation oder für Ammoniumkation steht, in Anwesenheit oder ohne Zusatz eines inerten organischen Lösungsmittels in ein Imidazolon VII

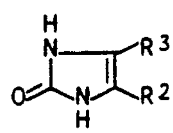

VII

überführt, und dieses anschließend in Substanz oder in einem inerten organischen Lösungsmittel in Gegenwart oder Abwesenheit einer Base mit einem Chlorierungsmittel zu Ia umsetzt.

3. Verfahren nach Anspruch 2, dadurch gekennzeichnet, daß man die Reduktion von IV zu V in einem inerten Lösungsmittel in Gegenwart eines Hydrierkatalysators mit Wasserstoff vornimmt.

4. Verfahren zur Herstellung der Verbindungen Ib gemäß Anspruch 1, dadurch gekennzeichnet, daß man ein 2-Chlorimidazolderivat Ia mit einem elektrophilen Reagens VIII,
$R^1$-Y VIII
worin Y Halogen, eine Sulfonat- oder eine Sulfatgruppe bedeutet, zu Ib umsetzt.

5. Herbizides Mittel, enthaltend ein substituiertes Chlorimidazolderivat Ia und/oder Ib gemäß Anspruch 1 und inerte Zusatzstoffe.

6. Herbizides Mittel gemäß Anspruch 5, enthaltend ein substituiertes Chlorimidazolderivat Ia und/oder Ib

und weitere wirksame Bestandteile.

7. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines substituierten Chlorimidazolderivates Ia und/oder Ib gemäß Anspruch 1 behandelt.

8. Verwendung der substituierten Chlorimidazolderivate Ia und/oder Ib gemäß Anspruch 1 als Herbizide.

Patentansprüche für folgenden Vertragsstaat: ES

1. Verfahren zur Herstellung der 2-Chlorimidazolderivate Ia

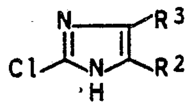

Ia

in denen die Substituenten folgende Bedeutung haben:
$R^2$ eine $C_1$-$C_4$-Alkylgruppe, eine $C_3$-$C_6$-Alkenylgruppe, oder eine $C_3$-$C_6$-Alkinylgruppe, wobei diese Gruppen ein bis drei der folgenden Reste tragen können: Halogen, $C_1$-$C_4$-Alkoxy und/oder $C_1$-$C_4$-Alkylthio;
$R^3$ -CN; $-CO_2R^4$; $-CONR^5R^6$ oder $-COR^7$;
$R^4$, $R^5$, $R^6$ und $R^7$ Wasserstoff;
$C_1$-$C_{10}$-Alkyl, welche einfach durch $C_3$-$C_{10}$-Cycloalkyl, $C_1$-$C_4$-Alkoxy, $C_1$-$C_4$-Alkylthio oder Phenyl substituiert sein kann, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
$C_3$-$C_{10}$-Cycloalkyl, welches ein- bis dreifach durch $C_1$-$C_4$-Alkyl substituiert sein kann;
$C_3$-$C_{10}$-Alkenyl;
$C_3$- $C_{10}$-Alkinyl oder Phenyl, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
sowie deren umweltverträglichen Salze, dadurch gekennzeichnet, daß man eine Carbonylverbindung II

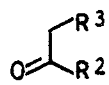

II

in an sich bekannter Weise ohne Isolierung der Zwischenstufen nacheinander in einer wäßrigen Säure zuerst mit einem Nitrit III,
$A^{\oplus}NO_2^{\ominus}$ III
worin $A^{\oplus}$ ein Alkalimetallion bedeutet, zu IV nitrosiert,

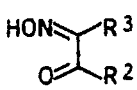

IV

danach zu V reduziert,

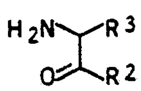

V

und direkt mit einem Cyanat VI,
$B^{\oplus}OCN^{\ominus}$ VI
worin $B^{\oplus}$ für ein Alkalimetallkation oder für Ammoniumkation steht, in Anwesenheit oder ohne Zusatz eines inerten organischen Lösungsmittels in ein Imidazolon VII

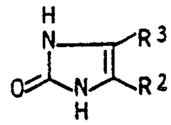

VII

überführt, und dieses anschließend in Substanz oder in einem inerten organischen Lösungsmittel in Gegenwart oder Abwesenheit einer Base mit einem Chlorierungsmittel zu Ia umsetzt.

2. Verfahren nach Anspruch 1, dadurch gekennzeichnet, daß man die Reduktion von IV zu V in einem inerten Lösungsmittel in Gegenwart eines Hydrierkatalysators mit Wasserstoff vornimmt.

3. Verfahren zur Herstellung der 2-Chlorimidazolderivat Ib

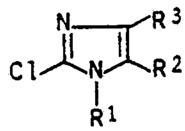

Ib

in denen die Substituenten folgende Bedeutung haben:
$R^1$ eine $C_1$-$C_4$-Alkylgruppe, eine $C_3$-$C_6$-Alkenylgruppe oder eine $C_3$-$C_6$-Alkinylgruppe, wobei diese Gruppen ein bis fünf Halogenatome und/oder einen Phenylrest tragen können;
$R^2$ eine $C_1$-$C_4$-Alkylgruppe, eine $C_3$-$C_6$-Alkenylgruppe, oder eine $C_3$-$C_6$-Alkinylgruppe, wobei diese Gruppen ein bis drei der folgenden Reste tragen können: Halogen, $C_1$-$C_4$-Alkoxy und/oder $C_1$-$C_4$-Alkylthio;
$R^3$ -CN; $-CO_2R^4$; $-CONR^5R^6$ oder $-COR^7$;
$R^4$, $R^5$, $R^6$ und $R^7$ Wasserstoff;
$C_1$-$C_{10}$-Alkyl, welche einfach durch $C_3$-$C_{10}$-Cycloalkyl, $C_1$-$C_4$-Alkoxy, $C_1$-$C_4$-Alkylthio oder Phenyl substituiert sein kann, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
$C_3$-$C_{10}$-Cycloalkyl, welches ein- bis dreifach durch $C_1$-$C_4$-Alkyl substituiert sein kann;
$C_3$-$C_{10}$-Alkenyl;
$C_3$- $C_{10}$-Alkinyl oder Phenyl, wobei der aromatische Ring seinerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio substituiert sein kann;
sowie deren umweltverträglichen Salze, dadurch gekennzeichnet, daß man ein 2-Chlorimidazolderivat Ia gemäß Anspruch 1 mit einem elektrophilen Reagens VIII,
$R^1$-Y VIII
worin Y Halogen, eine Sulfonat- oder eine Sulfatgruppe bedeutet, zu Ib umsetzt.

4. Herbizides Mittel, enthaltend ein substituiertes Chlorimidazolderivat Ia und/oder Ib gemäß Anspruch 1 und 2 und inerte Zusatzstoffe.

5. Herbizides Mittel gemäß Anspruch 4, enthaltend ein substituiertes Chlorimidazolderivat Ia und/oder Ib und weitere wirksame Bestandteile.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines substituierten Chlorimidazolderivates Ia und/oder Ib gemäß Anspruch 1 und 2 behandelt.

7. Verwendung der substituierten Chlorimidazolderivate Ia und/oder Ib gemäß Anspruch 1 und 2 als Herbizide.

Europäisches Patentamt

# EUROPÄISCHER RECHERCHENBERICHT

Nummer der Anmeldung

EP 89115082.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 5) |
|---|---|---|---|
| A | DE - A1 - 3 504 677 (A.NATTERMANN & CIE GMBH) * Ansprüche 1,5; Seite 16, Zeile 24 - Seite 17, Zeile 12 * -- | 1 | C 07 D 233/68<br>A 01 N 43/50 |
| A | CHEMICAL ABSTRACTS, Band 103, Nr. 25, 23. Dezember 1985, Columbus, Ohio, USA YAMANOUCHI PHARMACEUTICAL "Imidazole nucleoside derivatives" Seite 939, Spalte 1, Zusammenfassung-Nr. 215 736q & Jpn. Kokai Tokyo Koho JP 60 109 595 (85 109 595) -- | 1 | |
| A | CHEMICAL ABSTRACTS, Band 92, Nr. 11, 17. März 1980, Columbus, Ohio, USA SINYAKOV, A.N. et al. "Trans-halogenation in chloroethynyl derivatives of nitrogen-containing heterocycles" Seite 585, Spalte 1, Zusammenfassung-Nr. 94 318j & Izv. Akad. Nauk SSSR, Ser. Khim. 1979, (10), 2306-12 -- | 1 | RECHERCHIERTE SACHGEBIETE (Int. Cl 5)<br>C 07 D 233/00<br>A 01 N 43/00 |
| A | GB - A1 - 1 197 101 (AGRIPAT S.A.) * Anspruch 1 * ---- | 1,5-8 | |

Der vorliegende Recherchenbericht wurde für alle Patentansprüche erstellt.

| Recherchenort | Abschlußdatum der Recherche | Prüfer |
|---|---|---|
| WIEN | 13-11-1989 | BRUS |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument